# EUROPEAN PATENT APPLICATION

(11) **EP 4 628 137 A1**
(43) Date of publication of application: **08.10.2025**
(21) Application number: 24168805.0
(22) Date of filing: 05.04.2024
(51) Int. Cl.: A61M 25/00

(54) **METHOD AND SYSTEM FOR MANUFACTURING A PROTECTIVE SLEEVE ARRANGEMENT FOR A URINARY CATHETER**

(71) Applicant: Wellspect AB, 431 21 Mölndal (SE)
(72) Inventor: CARLANDER, Rolf, 423 63 Torslanda (SE)
(74) Representative: Aldridge, Henry Alexander

(57) **Abstract**

The present disclosure relates to a method and a system for manufacturing a protective sleeve arrangement for a urinary catheter. The method comprises providing a first holder part a second holder part, each having a channel extending therethrough. The method further comprises arranging the first and second holder part on a mandrel such that the mandrel extends through the channels and forming a tubular sleeve, the tubular sleeve being flexible and connecting the tubular sleeve to the first holder part and to the second holder part. The method further comprises moving the first holder part along the mandrel, towards the second holder part, releasably connecting the first holder part to the second holder part to form the protective sleeve arrangement; and removing the protective sleeve arrangement from the mandrel.

## Description

### TECHNICAL FIELD OF THE INVENTION

The present invention relates to a method and system for manufacturing a protective sleeve arrangement for a urinary catheter.

### BACKGROUND OF THE INVENTION

Urinary catheters are widely used for intermittent catheterization. In intermittent catheterization, the urinary catheter is inserted into the bladder via the urethra, urine is discharged via the catheter and the catheter is then removed. Urinary catheters typically comprise a hollow catheter shaft which is adapted for insertion through the urethra and a connector, which is to remain outside the urethra, and allow the urine to exit from the hollow catheter shaft.

Many types of urinary catheters feature a catheter shaft which is coated with a hydrophilic coating that becomes slippery when wetted, to make insertion easier and more comfortable. A urinary catheter provided with a hydrophilic coating may be provided in a ready-to-use state inside an external package. To achieve this, the external package contains a wetting fluid which is in contact with the catheter shaft to keep the hydrophilic coating constantly wetted (and thereby slippery) so as to be ready for immediate use upon removal from the external package. Alternatively, some external packages are configured to store the urinary catheter in substantially dry state but further comprises a wetting element which is configured to wet the hydrophilic coating of the catheter shaft when the urinary catheter is removed from the external package. As a further example, there are urinary catheter products where a substantially dry urinary catheter is provided in an external package together with a sachet containing a wetting fluid, whereby a user can open the sachet and manually wet the catheter shaft with fluid from the sachet.

A benefit of the above catheter products is that the user does not need separate access to a wetting fluid (e.g. water) making it possible for users to perform catheterization at various locations.

However, there are also urinary catheters with a hydrophilic coating which are provided in a completely dry state inside a simple external package without a wetting fluid provided in the package, a sachet with wetting fluid, a wetting element or the like. With these products the user opens the package and wets the catheter shaft with e.g. tap water to make it ready for use. For example, the user adds tap water directly into the package after opening the package, to thereby wet the catheter shaft.

However, there is a problem in that many users have difficulties in avoiding touching the catheter shaft, prior to inserting the catheter through the urethra or after the catheter has been removed from the urethra. For example, to keep the catheter shaft sterile before use it is crucial that the catheter shaft does not contact the user directly or any other objects. Similarly, when the catheter is removed after use, users want to avoid touching the catheter shaft since this part may be contaminated by urine.

Performing urinary self-catheterization without touching the catheter shaft is therefore challenging, especially if the user has reduced dexterity. Holding the urinary catheter only by the connector during use means that the user will not have to touch the catheter shaft, but this usage of the catheter may still be very difficult in some circumstances.

To overcome these problems, various types of protectors and gripping devices have been proposed that can fit over the catheter shaft allowing the user to hold the catheter via the protector or gripping device without directly touching the catheter shaft. The protector or gripping device is preferably also displaceable along the catheter shaft to allow users to insert the catheter shaft into the urethra without the protector or gripping element getting in the way.

In WO 2005/092418 a catheter package that also acts as a protector is shown. The protector comprises two rigid cylinders connected with a tubular plastic film, wherein the catheter is placed inside the tubular plastic film.

In EP 2 500 056 a gripping element for urinary catheters is shown. The gripping element can slide along the catheter shaft and be used to grip the catheter shaft by squeezing the gripping element.

However, while different types of protectors and gripping elements exist, these are often challenging to manufacture due to their intricate design.

### SUMMARY OF THE INVENTION

In view of the drawbacks of existing solutions there is a need for an improved method for manufacturing a protective sleeve arrangement, i.e. a protector, for a urinary catheter, a urinary catheter including such a protective sleeve arrangement, and an improved manufacturing system. It is the purpose of the present disclosure to present such an improved manufacturing method and system.

According to a first aspect of the present invention there is provided a method for manufacturing a protective sleeve arrangement for a urinary catheter. The method comprises, providing a first holder part having a channel extending therethrough and providing a second holder part having a channel extending therethrough. The method further comprises forming a tubular sleeve (at least partially around the first and second holder part) the tubular sleeve being flexible and connecting the tubular sleeve to the first holder part and to the second holder part. The method further comprises arranging the first and second holder part on a mandrel such that the mandrel extends through the channels of the first and second holder part and moving the first holder part along the mandrel, towards the second holder part, releasably connecting the first holder part to the second holder part to form the protective sleeve arrangement and removing the protective sleeve arrangement from the mandrel.

A mandrel is an elongated shaft. The mandrel may be substantially straight and rigid. For example, the mandrel is made from a hard material such as metal.

By utilization of a mandrel it is possible to manufacture protective sleeve arrangements in an efficient manner well suited for mass production. The mandrel prohibits the flexible tubular sleeve from becoming accidentally trapped between the two holder parts when the holder parts are brought together and (releasably) connected. This means that the risk for damaging the protective sleeve is reduced.

Additionally, the mandrel provides more controlled compactization (e.g. pleating or folding) of the tubular sleeve since the tubular sleeve, while it gets compacted by the first holder part moving towards the second holder, is supported by the outside of the mandrel.

A further benefit is that the mandrel contributes to fixating the positions of the holder parts allowing the flexible sleeve to be connected to the holder parts in precise and repeatable manner.

Specifically, the disclosed manufacturing method enables production of the protective sleeve arrangement to be made in a speedy and cost-efficient manner, thereby lowering the overall production costs for the protective sleeve arrangement. At the same time, the production is reliable and controllable, leading to a high quality in the end product and lower amount of scrap.

In some implementations, the first and second holder part are already arranged on the mandrel when the tubular sleeve is formed. Accordingly, the steps of forming the tubular sleeve, and moving the first holder part towards the second holder part may both be performed with the holder parts arranged on a same mandrel.

Alternatively, it is envisaged that the tubular sleeve is formed and attached to the holder parts separately whereby the tubular sleeve, and the first and second holder parts coupled thereto, are subsequently arranged on the mandrel, prior to moving the first holder part towards the second holder part.

In some implementations when the first and second holder parts are arranged on a mandrel prior to forming the tubular sleeve the step of forming a tubular sleeve comprises providing two flexible sheet layers on opposite sides of the mandrel, each flexible sheet layer extending between the first and second holder part and joining the two flexible sheet layers together along two joining lines to form the tubular sleeve, the joining lines extending substantially parallel to the mandrel. The step of forming a tubular sleeve further comprises attaching respective end portions of the tubular sleeve to the first holder part and the second holder part, respectively, at circumferential attachment regions.

It is however envisaged that a similar method of forming the tubular sleeve may be performed even if the holder parts are not already arranged on the mandrel.

By forming the tubular sleeve by joining two, initially separate, flexible sheet layers, the method can be performed in a more efficient and rapid manner. Compared to e.g. separate provision of a tubular sleeve, which must be threaded over the holder parts, the step of joining the two flexible sheets around the holder parts directly allows for a manufacturing method better suited for materials provided on a roll. Additionally, the process results in very little waste material and circumvents any issues with a separately prepared tubular sleeve, such as the problem of keeping a tubular sleeve open to receive the holder parts even when the tubular sleeve is made of a material which crimples and adheres to itself by e.g. electrostatic attraction.

The circumferential attachment regions attach the tubular sleeve to the holder parts, such that the holder parts and the tubular sleeve form a protective sleeve arrangement.

Each circumferential attachment region extends over at least a portion of the outer circumference of the holder parts. Optionally, each circumferential attachment region individually or together with other circumferential attachment regions or attachment points covers a majority of, or the entirety, of the outer circumference of each holder part.

In some implementations, the method further comprises attaching respective end portions of the flexible sheets to the first holder part and second holder part, respectively, at attachment points prior to joining the two flexible sheet layers to each other.

This applies both if the holder parts are already arranged on the mandrel when tubular sleeve is formed or if the holder parts are separate from the mandrel when the tubular sleeve is formed.

With the attachment points, the flexible sheets are held at the correct place when they are joined together to form the tubular sleeve. This facilitates accurate formation of the joining lines. The attachment points may be smaller compared to the circumferential attachment regions. For example, the attachment points are point-like and the circumferential attachment regions are elongated, stretching around a portion of the outer circumference of the respective holder part.

In some implementations, the attachment points and circumferential attachment regions are circumferentially separated from each other on each holder parts.

Thus, the circumferential attachment regions and/or the attachment points cooperate in attaching the tubular sleeve to the holder parts at multiple locations which enables the tubular sleeve to be reliantly attached to each holder part. The circumferential attachment regions and/or attachment further ensure that the tubular sleeve is kept tightly around each holder part so as to avoid formation of excessive folds at the holder parts which may make these more difficult to grip. For example, it is generally desirable to keep the tubular sleeve close to the holder portions and/or away from the channel extending therethrough since this may otherwise make the holder parts more difficult to grip or risks trapping the tubular sleeve inside the channel of the holder parts.

In some implementations, the attachment points and the circumferential attachment region overlaps at least partially. For example, the attachment point is provided mainly to hold the flexible sheets in place while forming the elongated joining lines whereas the circumferential attachment region is formed later to hold the tubular sleeve close to the respective holder part. As will be described below, it is further envisaged that multiple circumferential attachment regions are formed in an overlapping or non-overlapping manner to hold the tubular sleeve against the holder parts.

In some implementations, the first holder part comprises a first connecting feature and the second holder part comprises a second connecting feature, configured to engage the first connecting feature and the step of connecting the first holder part and second holder part comprises engaging the first connecting feature with the second connecting feature.

The connecting features of the holder parts enable the holder parts to releasably but reliantly attach to each other. The connecting features may be configured to releasably connect the holder parts together when they are brought together along an axial direction centered in the channel of each holder part. The connecting features allow the holder parts to form a compact protective sleeve arrangement which can be in an extended state, when the connecting features are disengaged and the holder parts moved apart, or a compacted state, when the connecting features engage each other.

In some implementations, the tubular sleeve, when connected to the first and second holder part, covers the first and second connecting feature, enabling the first and second connecting feature to connect inside the tubular sleeve.

Accordingly, the tubular sleeve may be stored in the compacted state on the outside of the holder parts. By arranging the tubular sleeve on the outside of the holder parts the protective sleeve arrangement may be easier to use and the risk of the tubular sleeve getting trapped between the catheter and either of the holder parts is reduced.

In some implementations the channel of the first holder part is configured to receive at least a part of the second holder part, and the step of connecting the first holder part to the second holder part comprises receiving the second holder part at least partially inside the first holder part.

Hereby, the holder parts when arranged in the compacted state may have a total axial extension smaller than the sum of the axial extension of the holder parts when these are separated from each other. This makes the protective sleeve arrangement even more compact when it is in the compacted state. The connecting features (if present) may be configured and arranged to allow the second holder part to be received inside the first holder part and connect the two holder parts, when the second holder part is received inside the first holder part.

In some implementations, the mandrel comprises a first portion with a first cross-sectional dimension and a second portion with a second cross-sectional dimension, wherein the first cross-sectional dimension is larger than the second cross-sectional dimension. Wherein the step of arranging the first and second holder part on the mandrel comprises arranging the first holder part on the first portion and arranging the second holder part on the second portion.

A mandrel with a varying cross-sectional dimension has been found to facilitate efficient manufacturing since the tubular sleeve gets compacted in a more controlled manner when the holder parts are connected together.

For example, the mandrel may comprise a transition portion where the cross-sectional dimension changes from the first cross-sectional dimension to the second cross-sectional dimension, and the step of arranging the second holder part on the mandrel comprises arranging the second holder part adjacent to, or preferably abutting, the transition portion.

Since the second holder part is arranged up against the transition portion the tubular sleeve gets pushed off the first portion of the mandrel as the first holder part is moved towards the second holder part. The tubular sleeve will then land on the second holder part and get compacted in a controlled manner.

In some implementations, the first cross-sectional dimension is larger than an inner cross-sectional dimension of the channel in the second holder part.

This prohibits the second holder part from sliding onto the first portion of the mandrel, allowing the position of the second part to be restrained during manufacturing.

In some implementations, an outer cross-sectional dimension of the second holder part is equal to or smaller than the first cross-sectional dimension of the mandrel.

By providing a first portion of the mandrel which has a cross-sectional dimension which exceeds the cross-sectional dimension of the second holder part, the tubular sleeve can easily slide off the first portion of the mandrel and onto the second holder part.

In some implementations, the mandrel comprises one of an indentation and a protrusion, and wherein the channel of the first holder part comprises the other one of an indentation and a protrusion configured to engage the indentation or protrusion of the mandrel so as to rotationally lock the first holder part relative the mandrel.

The protrusion and indentation form a locking mechanism which rotationally locks the first holder part to the mandrel. Rotational locking prohibits the tubular sleeve from twisting during compactization which further facilitates forming a compact protective sleeve arrangement. Additionally or alternatively, a similar arrangement is provided for the mandrel and the second holder as well. That is, the mandrel may comprise one of an indentation and a protrusion, and wherein the channel of the second holder part comprises the other one of an indentation and a protrusion configured to engage the indentation or protrusion of the mandrel so as to rotationally lock the second holder part relative the mandrel.

The protrusion and/or indentation provided on one of the holder parts may form part of the connecting feature.

According to a second aspect of the invention, there is provided a method for manufacturing a catheter with a protective sleeve arrangement. The method comprises manufacturing a protective sleeve arrangement in accordance with the first aspect and providing a urinary catheter comprising a catheter tube having an insertion end and a connector attached to the end of the catheter tube that is opposite the insertion end. The method further comprises inserting the catheter tube with the insertion end first through the channels of the first and second holder part of the protective sleeve arrangement and connecting the first or second holder part to the connector.

Using this method a urinary catheter provided with the protective sleeve arrangement in a compacted state can be provided. When a user is to use the urinary catheter, the user may choose to extend the protective sleeve arrangement or leave it in the compacted state. Additionally, in some implementations the first or second holder part is releasably connected to the connector whereby the user further has the choice of removing the compacted sleeve arrangement entirely. In the compacted state, the protective sleeve arrangement is small and compact whereby a urinary catheter equipped with a protective sleeve arrangement is not much heavier, bulkier or larger than a regular urinary catheter.

In some implementations of the second aspect the method further comprises placing the catheter with the protective sleeve arrangement in an outer package.

The outer package may further contain a sachet with wetting fluid, for wetting the catheter shaft, or a wetting element which wets the catheter shaft upon removal from the external package. The urinary catheter may also be provided in the external package in direct contact with the wetting fluid such that the urinary catheter is already wetted and in a ready-to-use state. Alternatively, the outer package does not comprise any wetting fluid as is the case for dry products.

According to a third aspect of the invention, there is provided a manufacturing system for manufacturing a protective sleeve arrangement for a urinary catheter. The manufacturing system comprises a mandrel and a holder part arrangement subsystem, configured to arrange a first and second holder part on the mandrel, wherein each holder part comprises a channel through which the mandrel is inserted. The manufacturing system further comprises a tubular sleeve forming subsystem, configured to, , form a tubular sleeve, the tubular sleeve being flexible and attached to the first holder part and the second holder part, respectively, so as to

(flexibly) couple these parts together and an actuator subsystem configured to slide the first holder part along the mandrel to releasably connect the first holder part with the second holder part so as to form a protective sleeve arrangement, and to remove the protective sleeve arrangement from the mandrel.

The sleeve forming subsystem may be configured to form the tubular sleeve prior to the first and second holder part are arranged on the mandrel or while the first and second holder parts are arranged on the mandrel.

The manufacturing system enables completely automatic manufacturing of protective sleeve arrangements. In some implementations, the manufacturing system comprises a mandrel conveyor, configured to move a plurality of mandrels that move between the different subsystems. When a protective sleeve package has been manufactured and removed from a mandrel the mandrel can be reused. Accordingly, the manufacturing system is well suited for efficient, high-volume, production.

The invention according to the second and third aspect features the same or equivalent benefits as the invention according to the first aspect. Any functions described in relation to the method, may have corresponding features in a manufacturing system and vice versa.

### BRIEF DESCRIPTION OF THE DRAWINGS

The present invention will be described in more detail with reference to the appended drawings, showing currently preferred embodiments of the invention.
Figure 1 shows a side view of the first and second holder parts according to some implementations.
Figure 2 shows a side view of two holder parts flexibly coupled together by a tubular sleeve so as to form a protective sleeve arrangement, according to some implementations.
Figure 3a-b show a side view of a protective sleeve arrangement in its extended state and compacted state, respectively.
Figures 4a-f illustrate the steps performed in a manufacturing method according to some implementations.
Figure 5a is a flowchart illustrating a method for manufacturing a protective sleeve arrangement according to some implementations.
Figure 5b is a flowchart illustrating a method for forming a tubular sleeve, according to some implementations.
Figure 6 is a perspective view of the two holder parts arranged on a mandrel according to some implementations.
Figures 7a-e illustrate how the tubular sleeve is formed, according to some implementations.
Figures 8a-c illustrate how a protective sleeve arrangement can be attached to a urinary catheter, and extended during use of the urinary catheter, according to some implementations.
Figure 9a-g are top-views of stations in a manufacturing system, illustrating the steps performed by the manufacturing system while forming a tubular sleeve around a mandrel, according to some implementations.

### DETAILED DESCRIPTION OF CURRENTLY PREFERRED EMBODIMENTS

In the following detailed description, preferred embodiments of the invention will be described. However, it is to be understood that features of the different embodiments are exchangeable between the embodiments and may be combined in different ways, unless anything else is specifically indicated. For example, different types of connecting features provided on each holder part will be discussed but it is understood that even though a specific connecting feature is shown on the first holder part to interact with a specific connecting feature on the second holder part the connecting features could also be reversed.

Additionally, in the many of the described embodiments the tubular sleeve is formed while the first and second holder part are already arranged on the mandrel. By forming the tubular sleeve when the first and second holder part are already arranged on the mandrel it is e.g. possible to perform most of the manufacturing process with the holder parts arranged on the same mandrel, which may facilitate manufacturing efficiency. However, it is also possible to form the tubular sleeve around the holder parts (and attach it to the holder parts) prior to arranging the holder parts (coupled by the tubular sleeve) on the mandrel. It may also, for the sake of clarity, be noted that the dimensions of certain components illustrated in the drawings may differ from the corresponding dimensions in real-life implementations of the invention.

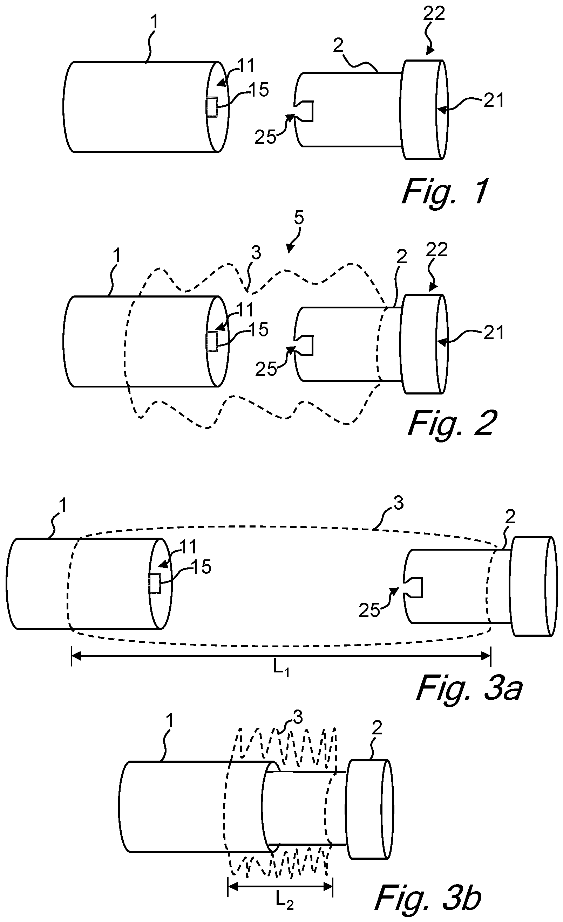

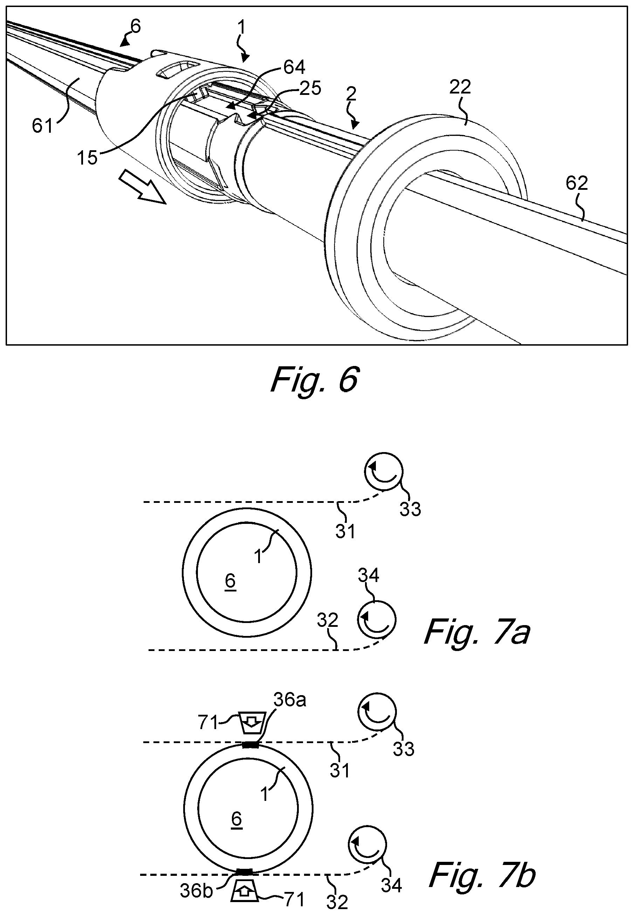

Fig. 1 illustrates a first holder part 1 and a second holder part 2 according to some implementations. Each holder part 1, 2 comprises a respective channel 11, 21 extending through the holder part 1, 2. Accordingly, each holder part 1, 2 is hollow, with a channel therethrough, and may be generally tube shaped. In some implementations, one of the holder parts 1, 2 is configured to be (optionally releasably) attached to the outside of a connector on a urinary catheter (see e.g. fig. 7a and 7b) and the holder parts 1, 2 may also be configured to be connected together. The holder part 1, 2 which is configured to be connected to the connector of a urinary catheter may be referred to as a distal holder part since it is distant from the user when he or she uses the urinary catheter for self-catheterization. Consequently, the other holder part 1, 2 may be referred to as the proximal holder part since it is closer to the user when he or she uses the urinary catheter for self-catheterization.

Without loss of generality, it will be assumed that the first holder part 1 is configured to be (optionally releasably) attached to the connector of a urinary catheter. However, it is understood that it may also be the second holder part 2 which is configured to be (optionally releasably) attached to the connector of a urinary catheter. It is also envisaged that both holder parts 1, 2 may be configured for (optionally releasably) attaching to the connector of a urinary catheter, allowing either one of the holder portions 1, 2 to be connected to the connector of a catheter as desired.

To make the first holder part 1 attachable to the connector on a urinary catheter, the first holder part 1 may comprise one or more connector connecting features which allows it to reliantly (and optionally releasably) attach to the outside of a connector on a urinary catheter. For example, the channel 11 of the first holder part may be provided with at least one of a recess and a protrusion which is configured to engage a corresponding recess or protrusion provided on the outside of the catheter connector. This allows the first holder portion 1 to be threaded onto the shaft of the urinary catheter and connected to the outside of the connector.

In one exemplary implementation, the channel 11 of the first holder part 1 is adapted to fit tightly around the connector to thereby hold the first holder part 1 to the connector. In another exemplary implementation, the channel 11 of the first holder part 1 comprises a circumferential protrusion running around the inside of the channel 11 so as to be attachable to a corresponding circumferential indentation on the connector.

Other connecting features may also be provided on the first holder part 1 to make it attachable to the connector of a urinary catheter. For example, the channel 11 of the first holder part 1 comprises threads configured to engage threads provided on the connector of the urinary catheter.

Additionally or alternatively, the first holder part 1 may comprise one or more connecting features configured to releasably engage corresponding connecting features on the second holder part 2. This allows the first and second holder part 1, 2 to be releasably connected together. For example, the first holder part 1 comprises a protrusion 15 inside its channel 11 wherein the protrusion 15 is configured to releasably engage an opening 25 in the second holder part 2. Additionally or alternatively, the protrusion 15 of the first holder part 1 may be configured to engage a recess in the second holder part 2. These are just a few, out of many, possible examples for connecting features that can be provided on the holder parts 1, 2 to allow the holder parts 1, 2 to be releasably connected. Other types of snap locks, friction locks or press lock arrangements can also be used.

In some implementations, the force required to disconnect the first and second holder part 1, 2 from each other is smaller than the force required to disconnect the first holder portion from the connector of the catheter. This ensures that upon use, the user will move the second holder part 2 along the catheter shaft, and not accidentally dislodge both the first and second holder part 1, 2 from the connector of the catheter, which although possible is seldom desired during normal use.

The first and second holder parts 1, 2 are both intended to allow the shaft of a urinary catheter to pass through their respective channels 11, 21. To this end, each channel 11, 21 of each holder part 1, 2 has a cross-sectional dimension that is larger than the outer diameter of the shaft of the urinary catheter with which the protective sleeve arrangement is intended to be used. For example, the cross-sectional dimension of each channel 11, 21 is at least 6 mm, at least 8 mm or at least 10 mm. Since most urinary catheter shafts have a circular cross-section the cross-sectional shape of the channels 11, 21 may also be circular. However, it is understood that the cross-sectional shape of the channels 11, 21 alternatively could be polygonal, oval, or elliptical as long as the cross-sectional shape is sized and adapted to allow passage of a urinary catheter shaft.

The first and second holder parts 1, 2 may have outer surfaces of various shapes. Fig. 1 shows an exemplary circular cylindrical outer surface shape for the first holder portion 1. The second holder part 2 in fig. 1 also features a circular cylindrical outer surface but further features a flange 22 giving the second holder part 2 a three-dimensional shape similar to a "mushroom" shape. The provision of a flange 22 may make the second holder part 2 easier to grab and/or provide a surface against which the tubular sleeve can be packed in the compacted state, as will be described below. The person skilled in the art will realize that many different outer shapes may be used for realizing the first and second holder part 1, 2. Generally, since the holder parts 1, 2 are to be used, and optionally packaged together with a urinary catheter, the holder parts 1, 2 may be small and light while at the same time at least the second holder part 2 is easy for a user to grip and move along the shaft of the urinary catheter.

The first and second holder parts 1, 2 may be made of a wide variety of materials. For example the holder parts are made of a polymer material, such as a plastic material. For instance, the holding parts 1, 2 may be made of a plastic material and manufactured in an injection molding process.

As another example, the holder parts 1, 2 are made of a biobased material such as paper. Since the holder parts 1, 2 typically will not be exposed to large amounts of urine, or exposed to urine for long periods of time, the holder parts 1, 2 can be realized of a large variety of materials, as long as they are rigid enough to realize the connection to each other and the connector.

In some implementations, at least the second holder part 2 is elastic, enabling the second holder part 2 to be deformed by a user squeezing the second holder part 2 between his or her fingers wherein the second holder part 2 returns to its original shape when the user releases the squeezing force. This allows the user, when the second part 2 is arranged on a urinary catheter, to grip the urinary catheter via the second holder 2 by squeezing the second holder part 2.

Turning to fig. 2, a protective sleeve arrangement 5 is shown schematically. The protective sleeve arrangement 5 comprises the first and second holder part 1, 2 as described above, and a tubular sleeve 3 coupling the first holder part 1 to second holder part 2. The tubular sleeve 3 may also be referred to as a compactable sleeve since it is configured to be compacted.

The tubular sleeve 3 is generally tube shaped when fully extended but can also be compacted such that it folds in on itself and forms a stacked structure.

In some implementations, the tubular sleeve 3 is made of a thin plastic material, such as plastic film. The first and second holder parts 1, 2 are made of a more rigid material compared to the tubular sleeve 3. The material of the tubular sleeve 3 may be plastic foil such as polyethylene (PE) or polypropylene (PP) or coated or non-coated paper. However, other materials, such as other cellulose based or paper based materials, are also feasible. The tubular sleeve 3 may be at least temporarily impermeable to liquids such as urine and/or contaminations from a user's fingers.

The tubular sleeve 3 is mainly used to cover the surface of the urinary catheter shaft immediately prior to, and after, the catheter has been used. Thereby the flexible sleeve 3 need not be long-term impermeable. However, the tubular sleeve 3 should preferably not be too permeable because moisture and contaminations should be prevented from permeating through the tubular sleeve 3. However, there is no need for complete long-term impermeability because it is only used while the urinary catheter is inserted, which normally only takes a few minutes. This means that many types of material are suitable for use as the tubular sleeve 3, because the only requirement except at least short-term impermeability for urine and/or contaminations is that the material has to be foldable or in other ways compactable into a compressed state and from there be extendable with relative ease.

In some implementations, the tubular sleeve 3 is transparent, allowing the user to see the catheter shaft through the sleeve, e.g. to confirm that there are no damages or foreign objects, such as dirt or dust, on the catheter shaft or whether the shaft is properly wetted, even when the tubular sleeve 3 extends over the catheter shaft. Alternatively, the flexible sleeve 3 is opaque.

Figs. 3a and 3b show the protective sleeve arrangement 5 in its extended state and compacted state, respectively. In fig. 3a the first and second holder part 1, 2 are disengaged from each other and pulled apart. This causes the tubular sleeve 3 to unfold and reach its extended length L₁. This state of the protective sleeve arrangement 5 is referred to as the extended state, as opposed to the compacted state shown in fig. 3b. In fig. 3b, the first and second holder parts 1, 2 have been brought together and connected, whereby the tubular sleeve 3 becomes compacted and rests on the outside of holder parts 1, 2. In fig. 3b, the compacted sleeve 3 only extends a compacted length L₂ wherein L₂ is smaller than L₁.

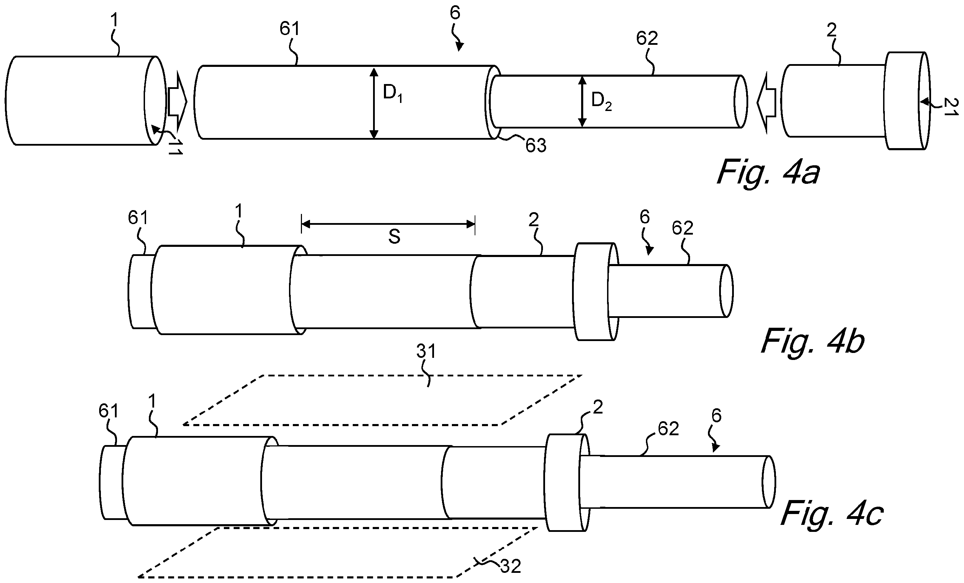

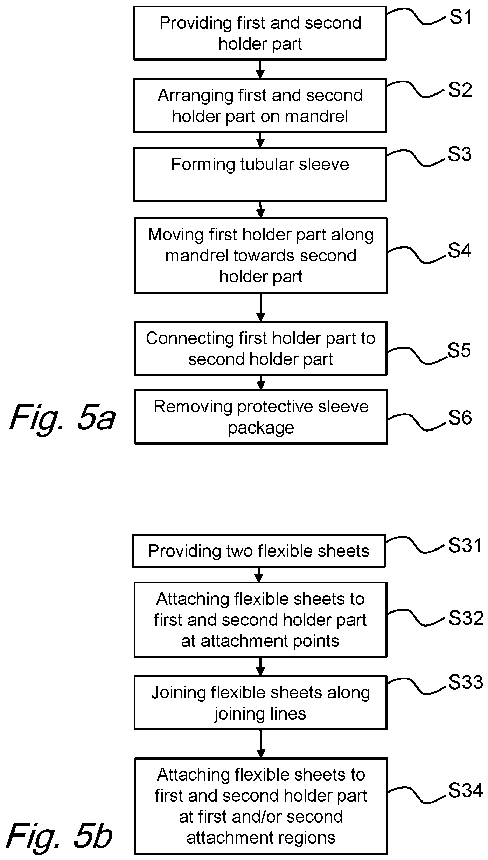

With reference to the flowchart in fig. 5a describing a method for manufacturing a protective sleeve arrangement and figs. 4a-f schematically illustrating the manufacturing steps, it will now be described how a protective sleeve arrangement 5 may be manufactured in an efficient process.

At step S 1, a first holder part 1 and a second holder part 2 are provided. The holder parts 1, 2 themselves may e.g. have been manufactured in a separate manufacturing process, e.g. in an injection molding process.

The first and second holder part 1, 2 are arranged on a mandrel 6 at step S2, as shown in fig. 4a and fig. 4b. The mandrel 6 is an elongated element configured to hold the first and second holder part 1, 2 by extending through the channels of each holder parts 1, 2. The mandrel 6 may e.g. be realized as a substantially cylindrical rod with a circular cross-section but other mandrel shapes are also possible, e.g. mandrels 6 with a cross-sectional shape that is polygonal, oval or elliptical are also envisaged. As will be described below, the mandrel 6 may comprise a varying cross-sectional dimension to make sure that the tubular sleeve is compacted correctly and does not become trapped between the holder parts 1, 2 during manufacturing.

In some implementations, the mandrel 6 comprises at least one protrusion and/or at least one recess that is configured to engage a corresponding at least one recess and/or at least one protrusion provided on one or more of the holder parts 1, 2 to facilitate retention and/or maintain desired orientation of the holder parts 1, 2 when they are arranged on the mandrel 6. For example, the mandrel 6 may comprise a recess forming a groove that extends along at least a part of the mandrel 6 or a protrusion forming a ridge that extends along at least a part of the mandrel 6, wherein the first and/or second holder part 1, 2 has the other one of a recess or protrusion adapted to interlock with the groove or ridge on the mandrel 6. This allows one or both of the holder parts 1, 2 to be rotatably locked relative the mandrel 6. Additionally, or alternatively, rotational locking may be achieved with a mandrel 6 having a non-circular cross-sectional shape which is configured to interlock with a channel having a non-circular cross-sectional shape in one or both of the holder parts 1, 2.

The holder parts 1, 2 are arranged on the mandrel 6 with a predetermined separation distance S, as seen in fig. 4b. The predetermined separation distance S may be set such that the first and second holder parts 1, 2 are separated by a distance corresponding to their separation when the protective sleeve arrangement is in its fully extended state. In this case, if the tubular sleeve is fully stretched when it is attached to the holder parts 1, 2 the separation distance S will dictate the fully extended length of the protective sleeve arrangement, wherein the separation distance S preferably is such that it covers a majority of the length of a urinary catheter. For example, the separation distance S may be slightly shorter than the compactable tube, allowing the compactable tube to at least partially overlap each holder part 1, 2.

For example, the separation distance S is between 280 mm and 410 mm. This means that the resulting protective sleeve arrangement will have a maximum length of between 300 mm and 430 mm which is suitable for covering the insertable length of most types of intermittent urinary catheters for males. The insertable length for female catheters is shorter, typically between 60 - 130 mm whereby the separation distance S may be adjusted accordingly, to manufacture a protective sleeve arrangement which is more suitable for protecting shorter catheters.

In some implementations, the tubular sleeve is partially compacted when it is attached to the holder parts 1,2 whereby the total length of the protective sleeve arrangement, when it is fully extended, is greater than the separation distance S when the holder parts 1, 2 are on the mandrel. To this end, the separation distance S may be shorter than the exemplary lengths given above while still enabling the protective sleeve arrangement to extend to cover the majority or entirety of a urinary catheter shaft, in the extend state.

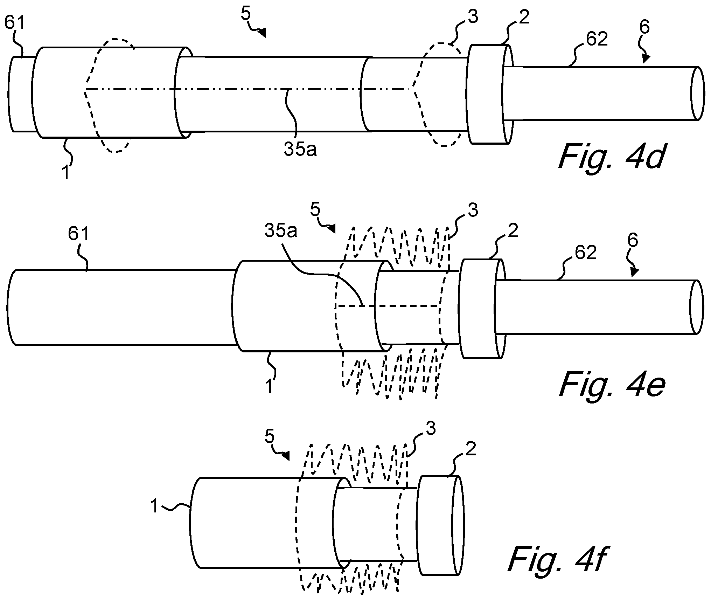

At step S3, a tubular sleeve 3 is provided over the mandrel 6 and at least a portion of each holder part 1, 2 as shown in fig. 4d. The tubular sleeve 3 is further attached to the outer surface of each holder part 1, 2 so that the channels 11, 21 through the holder parts 1, 2 together with the tubular sleeve 3 forms a single tubular structure.

The tubular sleeve 3 may be provided or formed over the mandrel 6 in various ways and different methods for forming the tubular sleeve will be described below, in connection to fig. 5b. In one exemplary embodiment, the tubular sleeve 3 is formed by providing two sheets 31, 32 on opposite sides of the mandrel 61 and joining the two sheets along a first joining line 35a and a second joining, the joining lines being on substantially opposite sides of the mandrel 6, as illustrated in fig. 4c and 4d.

The tubular sleeve 3 may be attached to each holder part 1, 2 along a portion of, a majority of, or the entire outer perimeter of each holder part 1, 2. While an attachment around the complete perimeter of each holder part 1, 2 may make the attachment between the tubular sleeve 3 and the respective holder part 1, 2 liquid tight, or even fluid tight, it is noted that the attachment between the tubular sleeve 3 and the respective holder part 1, 2 must not necessarily be liquid or fluid tight to still protect the catheter shaft and allow the user to grip the catheter shaft via the tubular sleeve 3. For example, the tubular sleeve 3 may be attached at separate attachment points distributed regularly or intermittently around the perimeter of the outer surface of each holder part 1, 2. As long as the spacing between the attachment points is smaller than the width of a finger, the user will be prevented from coming into contact with the catheter shaft. However, with a tubular sleeve having ends fitting relatively tightly over the holder parts, a single, or very few, attachment points may be used.

In some implementations, the tubular sleeve 3 is stretched while it is attached to each holder part 1, 2. This allows the attachment to be made more accurate.

In some implementations, step S3 may be performed prior to step S2. That is, the tubular sleeve 3 may be formed and attached to the holder parts 1, 2 prior to the holder parts 1, 2 (and the tubular sleeve 3) being arranged on the mandrel 6.

After steps S1-S3, the protective sleeve arrangement 5 is located on the mandrel 6 in an extended state as shown in fig. 4d. It is noted that this applies both if the tubular sleeve 3 was formed when the holder parts 1, 2 where already on the mandrel 6 or if the tubular sleeve 3 was formed and attached to the holder parts 1, 2 prior to these being arranged on the mandrel. The method then continues with step S4 involving moving the first holder part 1 towards the second holder part 2, along the mandrel 6. As the first holder part 1 moves towards the second holder part 2 the tubular sleeve 3 becomes compacted (e.g. pleated and/or generally folded) and forms an annular shape around the holder parts 1, 2 as shown in fig. 4e.

The first holder part 1 eventually reaches the second holder part 2 and at step S5 the first holder part 1 connects to the second holder part 2, as shown in fig. 4e. The connection between the two holder parts 1, 2, may be enabled by connecting features located on each holder part 1, 2, wherein the connecting features are configured to releasably engage each other.

Additionally or alternatively, one of the holder parts 1, 2 (in the embodiment of fig. 4e it is the first holder part 1) comprises a channel which is sized and adapted to fit over the other holder part 1, 2. The channel of one of the holder parts 1, 2 may be configured to fit tightly over the other holder part so as to thereby form a connection. When the two holder parts 1, 2 are connected, the tubular sleeve 3 is compacted and stored outside one or both holder parts 1, 2 in what is referred to as the compact state of the protective sleeve arrangement 5.

At step S6, the protective sleeve arrangement 5 is removed from the mandrel 6. For example, the displacement of the first holder part 1 towards the second holder part 2 is allowed to continue beyond connection of the first holder part to the second holder part 2, whereby the entire protective sleeve arrangement 5 may slide of the mandrel 6.

Fig. 4f shows the protective sleeve arrangement 5 after it has been removed from the mandrel 6.

During step S4 involving sliding the first holder part 1 towards the second holder part 2, it should preferably be ensured that the tubular sleeve 3 does not become trapped between the first holder part 1 and the mandrel 6 or between the first holder part 1 and the second holder part 2. To this end, the mandrel 6 may have a cross-sectional dimension which varies, as can be seen in figs. 4a-e.

In some implementations, the mandrel 6 has a first portion 61 and a second portion 62, wherein a cross-sectional dimension Di of the first portion 61 is larger than a cross-sectional dimension D₂ of the second portion 62. The first holder part 1 is arranged on the first portion 61 of the mandrel 6 during steps S2 and S3 and the second holder part 2 is arranged on the second portion of the mandrel 6 during steps S2 and S3. Hereby, since the first portion 61 has a larger cross-sectional dimension Di movement of the first holder part 1 towards the second holder part 2 during step S4 allows the tubular sleeve 3 to be continuously pushed off the larger first portion 61 of the mandrel 6 with a reduced risk of being trapped between the first and second holder part 1, 2 as the holder parts 1, 2 come closer together.

Even in cases where the tubular sleeve 3 is formed and attached to the holder parts 1, 2 prior to mounting the holder parts 1, 2 and the tubular sleeve 3 onto the mandrel 6 it is possible to thread the holder parts 1, 2 and the tubular sleeve 3 onto the mandrel 6. For example, by threading the first holder part 1 over the smaller second portion 62 first it is possible to arrange the first holder part 1 on the first portion 61 and the second holder part 62 on the second portion, even when these are flexibly coupled by the tubular sleeve.

In some examples, the transition from the first portion 61 of the mandrel 6 to the second portion of the mandrel 62 is abrupt, forming a stepwise transition 63. The second holder part 2 is preferably moved up to be in close proximity to the stepwise transition 63, or directly abutting the stepwise transition 63, so that there is little or no room for the tubular sleeve 3 to become trapped. Rather, the tubular sleeve 3 will slide off the first portion 61 of the mandrel 6 and land on the second holder part 2, located on the second portion 62 of the mandrel 6. However, instead of a stepwise transition, a gradual or tapering transition may be used.

A cross-sectional dimension of the outer surface of the second holder part 2 may be equal to, or smaller than, the cross-sectional dimension of first portion 61 of the mandrel 6.

In some implementations, a cross-sectional dimension of the channel of the first holder part 1 is larger than a cross-sectional dimension of the outer surface of second holder part 2, allowing the first holder part 1 to partially receive the second holder part 2 as it is moved over the mandrel 6.

Fig. 6 shows a perspective view of the first and second holder parts 1, 2 arranged on a mandrel 6 according to some implementations. In fig. 6, the tubular sleeve has been omitted to make the details of the holder parts 1, 2 and mandrel 6 clearly visible. The first holder part 1 is moved along the first portion 61 of the mandrel 6 towards the second holder part 2 arranged on the second portion 62 of the mandrel 6. In this embodiment, the first holder part 1 comprises a protrusion 15 in its channel that engages and slides in a groove 64 provided on the mandrel 6. The same protrusion 15 also forms a connecting feature that will connect by snapping into an aperture 25 formed in the second holder part 2 when the first holder part comes sufficiently close.

The second holder part 2 comprises a flange 22 protruding outwardly from the second holder part 2. The flange 22 may make it easier for a user to grab the second holder part 2 and disconnect it from the first holder part 1 during use and the flange may also act as a stopper that stops the tubular sleeve as it is pushed onto the second holder part 2 and thereby contributes to compacting the tubular sleeve on top of the holder parts 1, 2.

Turning to the flowchart in fig. 5b and figs. 7a-d, a method for forming the tubular sleeve 3 over the mandrel 6 and holder parts 1, 2 will now be described. While this embodiment relates to formation of the tubular sleeve 3 when the holder parts 1, 2 are arranged on a mandrel 6, it is understood that the same method may be applied if the holder parts 1, 2 are not on a mandrel 6. For example, arrangements other than a mandrel 6 may be used for holding the holder parts 1, 2 while forming the tubular sleeve 3.

At step S31 two flexible sheets 31, 32 are provided on substantially opposite sides of the mandrel 6 holding the two holder parts. Figs. 7a-d show cross-sectional views in a plane perpendicular to the mandrel 6 through the first holder part 1. While figs. 7a-d show how a tubular sleeve 3 may be formed around the first holder part 1 it is understood that an analogous or similar process is performed around the second holder part.

As seen in fig. 7a, step S31, involving providing two sheets 31, 32, may comprise unrolling a respective sheet 31, 32 from a respective roll 33, 34 of sheet material. The sheets 31, 32 will form the tubular sleeve 3 so the sheets are e.g. a plastic foil or some other material that can be used to form the tubular sleeve 3. In some implementations, the sheets 31, 32 are kept stretched on opposite sides of the mandrel 6 and holder part 1. By keeping the flexible sheets stretched 31, 32 it is easier to form precise attachment regions as will be described below.

While use of two separate sheets 31, 32 is preferred, the tubular sleeve may alternatively be formed by a single sheet, which is wrapped around the holder parts and mandrel. In yet another alternative, the sleeve may be provided by a material already in tubular form, such as a tubular foil.

To stretch the sheets 31, 32 the rolls 33, 34 may e.g. be releasable and lockable, enabling the sheets 31, 32 to be unrolled when the rolls 33, 34 are in the released state and then stretched while the rolls 33, 34 are in the locked state. Alternatively, a clamper (not shown) may be used to clamp each sheet 31, 32 downstream of the respective roll 33, 34. To perform the stretching, the free end of each sheet 31, 32 that is unrolled from the respective roll 33, 34 may held by a gripper (not shown).

In some implementations, the method then proceeds to step S32 schematically shown in fig. 7b involving attaching each flexible sheet 31, 32 to the first holder part 1 at respective attachment points 36a, 36b on the outer surface of the holder part 1. The attachment points 36a, 36b may be on substantially opposite sides of the first holder part 1. The attachment points 36a, 36b may be comparatively small compared to the circumferential attachment regions that will be described below. For example, the attachment points 36a, 36b involves attachment over an area shaped like a point, oval, circle, ellipse or polygonal on the outer surface of the first holder part 1.

Attachment at the attachment points may be realized using a variety of techniques. For example, the attachment may be realized by one or several of: ultrasonic welding, heat welding, laser welding, solvent welding, and gluing by provision of an adhesive. The shape of the resulting points 36a, 36b can be realized by selecting an appropriate size of the welding region (or optionally by moving the welding apparatus during welding) or by administering an appropriate amount of adhesive or solvent.

Additionally or alternatively, the attachment of the tubular sleeve to the holder parts 1, 2 at attachment points may be realized using mechanical attachment. For example, one or more elastic elements (such as clips) may be arranged over the one or more of the holder parts 1, 2 to trap the tubular sleeve between the elastic element and the holder part. For instance, one or both of the first and second holder parts 1, 2 may be provided with a circumferential protrusion over which the tubular sleeve is fitted whereafter a ring element is fitted over the circumferential protrusion to trap the tubular sleeve.

As another example of mechanical attachment, at least one of the holder parts 1, 2 is provided with spikes or claws that are configured to penetrate and reliantly hold the tubular sleeve. To avoid a user getting hurt by these features the spikes or claws may be comparatively soft and/or extend substantially parallel with the outer surface of the holder part 1, 2.

The attachment at the attachment points 36a, 36b may be permanent or temporary. For example, the attachment points 36a, 36b may be realized with a clamping element which during manufacturing clamps the sheets 31, 32 against the respective holder part 1, 2.

In the example shown in fig. 7b the attachment points are permanent and two heat welding heads 71 are brought down towards the first holder part 1 and forms the attachment points 36a, 36b that attaches the first and second flexible sheet 31, 32 to the first holder part 1 by forming a respective heat weld.

The method then proceeds from step S32 to step S33. However, step S32 is optional and may be omitted, the method according to some implementations therefore proceeds directly from step S31 to step S33.

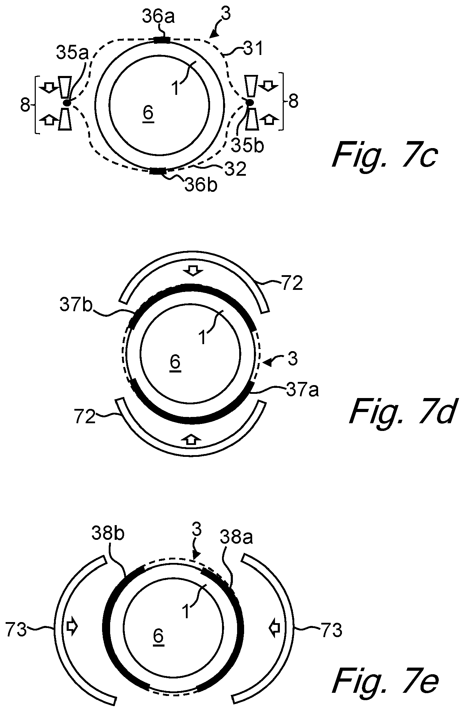

Step S33 involves forming elongated joining lines 35a, 35b, extending in an axial direction substantially parallel to the mandrel 6, which joins the two sheets 31, 33 together to form the tubular sleeve 3. Many different techniques could be used for joining the two sheets 31, 32 together along elongated joining lines 35a, 35b. In fig. 7c a two-part heat welding arrangement 8 is used to form each joining line 35a, 35b. In some implementations, one or both of the two-part heat welding arrangements 8 also cuts the sheets 31, 32 during the welding process so as to form a tubular sleeve 3 without residual material. The elongated joining lines may be continuous lines, preferably extending over the entire length of the tubular sleeve. However, alternatively, the elongated joining lines may be formed by shorter lines or point like welds arranged along a linear path.

A benefit with (at least temporarily or at least during step S33) providing the attachment points 36a, 36b at step S32 is that the sheets 31, 32 are held against the holder parts when attaching the sheets along the joining lines 35a, 35b. This may facilitate more accurate formation of the joining lines 35a, 35b.

The method then goes to step S34 involving forming at least one circumferential attachment region 37a, 37b on the first holder part 1. The circumferential attachment region 37a, 37b may be formed using the same techniques that are used for the attachment point 36a, 36b, (e.g. by means of heat welding, ultrasonic welding or mechanical attachment). In some implementations, the circumferential attachment region 37a, 37b is larger compared to the attachment point 36a, 36b. For example, one or more circumferential attachment regions 37a, 37b may be provided which together cover a majority of, or the entire, circumference of the first holder part 1.

In fig. 7d two circumferential attachment regions are provided. For example, each circumferential attachment region 37a, 37b covers about 120 degrees of the first holder part 1 and the circumferential attachment region 37a, 37b are provided on substantially opposite sides of the first holder part 1 and overlapping with the attachment points 36a, 36b, so as to together cover about 240 degrees of the circumferential perimeter of the first holder part 1. To realize these comparatively large circumferential attachment regions 37a, 37b large concave heat welding heads 72 may be used that follow the outer shape of the first holder part 1. Such larger circumferential attachment region may also, optionally, be used for realization of the attachment points.

The circumferential attachment region 37a, 37b may be located on opposite sides of the first holder part 1 and, for example, each circumferential attachment region 37a, 37b is overlapping with the attachment points 36a, 36b. For example, the attachment points 36a, 36b provide (e.g. temporary) retention that facilitates attaching the sheets 31, 32 along joining lines 35a, 35b and the circumferential attachment regions 37a, 37b form a larger attachment that contributes to making the tubular sleeve fluid or liquid tight and/or generally holding the tubular sleeve 3 to the holder parts 1, 2.

In some implementations, it is not necessary to form the attachment points 36a, 36b and the circumferential attachment regions 37a, 37b replaces the attachment points. However, to make it easier to form the elongated joining lines 35a, 35b the circumferential attachment regions 37a, 37b, if formed prior to the joining lines 35a, 35b, are preferably sized so as to leave the ends of the sheets 31, 32 free, to allow the joining lines 35a, 35b to be formed.

As another example, the center point of the circumferential attachment regions may be located about 90 degrees from the attachment points 36a, 36b as illustrated with circumferential attachment regions 38a, 38b in fig. 7e. With this type of circumferential attachment regions, the attachment points 36a, 36b may cooperate with the circumferential attachment regions 38a, 38b to cover the entire, or at least a majority of, the perimeter of the first holder part 1. For example, to cover the entire perimeter two attachment points 36a, 36b, each covering X degrees, centered on north and south points along the circumferential perimeter are provided in addition to two circumferential attachment regions 38a, 38b that each covers at least (180-X) degrees centered on west and east points along the circumferential perimeter. The circumferential attachment regions 38a, 38b may be formed using concave welding heads 73.

The attachment points 36a, 36b and the circumferential attachment regions 37a, 37b, 38a, 38b may be overlapping or non-overlapping.

Additionally, to completely cover the circumference of the first holder part 1 with an attachment region it is envisaged that both the opposite circumferential attachment regions 37a, 37b as shown in fig. 7d (hereby referred to as first circumferential attachment regions 37a, 37b) and the opposite circumferential attachment regions 38a, 38b (hereby referred to as second circumferential attachment regions 38a, 38b) as shown in fig. 7e are formed. The first circumferential attachment regions 37a, 37b may be provided overlapping with the attachment points 36a, 36b and with their respective center points displaced 90 degrees from the second circumferential attachment regions 38a, 38b whereby attachment is realized around the entire circumference of the first holder part 1.

It is further noted that instead of providing two first and/or second circumferential attachment regions 37a, 37b, 38a, 38b it is also envisaged that a single first and/or second circumferential attachment region 37a, 37b, 38a, 38b may be provided which e.g. runs around a majority of the perimeter of the first and/or second holder part 2.

The welding head 72, 73 used to form each of the circumferential attachment regions may be the same welding head whereby the holder part 1 and/or the welding head is reoriented (rotated) to form the different circumferential attachment regions 37a, 37b, 38a, 38b. Alternatively, different welding heads 72, 73 may be used. For example, a first pair of welding heads 72 form the substantially opposite first circumferential attachment regions 37a, 37b and another pair of welding heads 73 forms the second circumferential attachment regions 38a, 38b which are 90 degrees displaced from the first circumferential attachment regions 37a, 37b. The first pair of welding heads 72 may be arranged in a north-south arrangement and the second pair of welding heads 71 may be arranged in an east-west arrangement as shown in figs. 7d and 7e. Alternatively, the mandrel 6 carrying the holder parts 6 is rotated (e.g. 90 degrees) while moving from the first pair of welding heads 71 to the second pair of welding heads 72.

It is however not always necessary for the circumferential attachment regions 37a, 37b, 38a, 38b or the circumferential attachment regions 37a, 37b, 38a, 38b together with the attachment points 36a, 36b, to form a seal that completely covers the entirety of the outer perimeter of the first holder part 1. The attachment points and regions 36a, 36b, 37a, 37b, 38a, 38b are in part used to keep the tubular sleeve 3 attached to the first holder part 1 and to make sure that no loose ends of the tubular sleeve 3 extends out from the first holder part 1 to get in the way for the user when he or she uses a catheter with the protective sleeve arrangement. The attachment regions and points 36a, 36b, 37a, 37b, 38a, 38b are further used to create a seal between the holder parts and tubular sleeve 3 so that a user cannot come into contact with the catheter shaft. However, it is not required that the seal formed by the attachment regions and attachment points 36a, 36b, 37a, 37b, 38a, 38b is completely fluid tight or gas tight to prohibit the user from contacting the catheter shaft.

For example, the attachment regions and/or attachment points may be realized with welding dots spaced 1 mm apart, 2 mm apart or 3 mm apart which may not offer a fluid or liquid tight seal but is still sufficient to prohibit a user's finger from coming into contact with catheter shaft, between e.g. the first holder part 1 and the tubular sleeve 3. To this end, each attachment region and/or point 36a, 36b, 37a, 37b may be realized with a single continuous seal (e.g. a single continuous weld or string of adhesive) or each attachment region or point 36a, 36b, 37a, 37b, 38a, 38b may be realized with a plurality of spaced apart attachment points that e.g. form a dot matrix or a line of dots.

There are numerous benefits with forming the tubular sleeve using the method of fig. 5b and as illustrated in figs. 7a-e. Firstly, the utilization of two sheets 31, 32 means that the size of the tubular sleeve 3 can be adapted, by providing larger or smaller sheets 31, 32 which are joined together. Secondly, each sheet 31, 32 can the kept under tension, e.g. by being unrolled from a roll 33, 34, as the sheets 31, 32 are joined together, which facilitates making a straight joining line 35a, 35b compared to other methods, such as rolling a single flexible sheet around the holder portions.

As discussed above, it is possible to form the tubular sleeve 3 and attach it to the holder parts 1, 2 before the holder parts 1, 2 (coupled by the tubular sleeve 3) are arranged on the mandrel 6. In some implementations, one or more of the attachment points 36a, 36b and the (first and/or second) attachment regions 37a, 37b, 38a, 38b may be formed prior to arrangement on the mandrel 6 while one or more of the attachment points 36a, 36b and the (first and/or second) attachment regions 37a, 37b, 38a, 38b are be formed after arrangement on the mandrel 6.

While the method for forming the tubular sleeve 3 using two flexible sheets 31, 32 may be beneficial, other methods for forming the tubular sleeve 3 are also envisaged.

As one example, the tubular sleeve is provided separately and threaded over the mandrel holding the holder parts. After the tubular sleeve has been threaded over the mandrel and holder parts, the tubular sleeve is attached to the holder parts.

As another example, the tubular sleeve is created by providing a single sheet, rolling it around the mandrel and the holder parts, and then joining the single flexible sheet to itself to form the tubular sleeve that covers the mandrel and holder parts. The tubular sleeve can then be attached to the holder parts.

After the protective sleeve arrangement has been manufactured, it may be packaged individually and supplied to users as an individual product. A user may then use any urinary catheter and, when needed, further utilize a protective sleeve arrangement which the user can attach to the catheter by sliding the protective sleeve arrangement over the catheter shaft and connecting one of the holder parts to the connector.

In some implementations, the protective sleeve arrangement will instead be arranged on a catheter and provided to the user in a preinstalled state, either in the compacted state or in the extended state, on a urinary catheter.

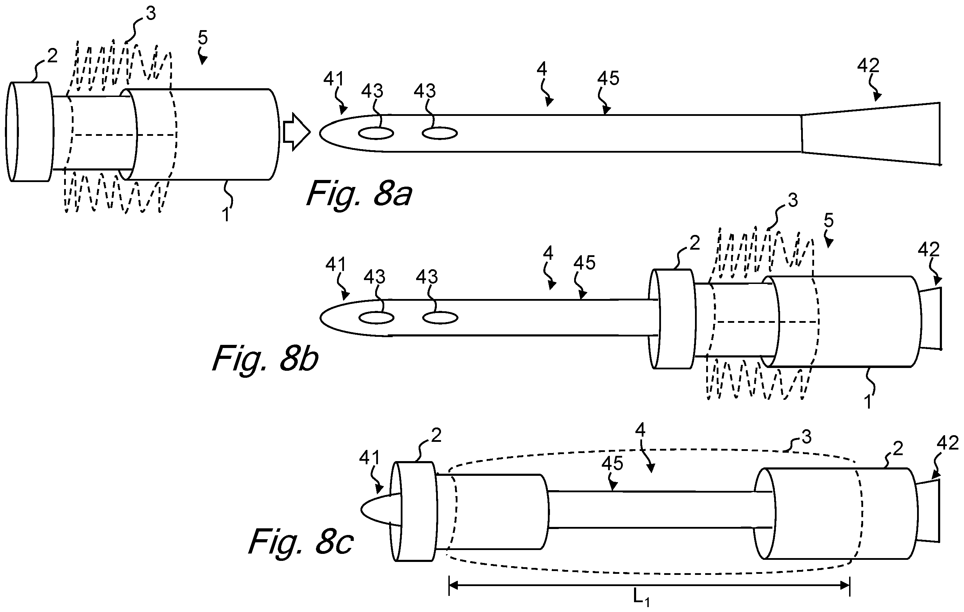

Accordingly, a method for manufacturing a urinary catheter may comprise first manufacturing a protective sleeve arrangement 5 as described above. Then, the method involves threading the protective sleeve arrangement 5 onto a urinary catheter, as shown in fig. 8a.

Fig. 8a shows a urinary catheter 4 comprising a catheter shaft 45 and a connector 42. The catheter shaft 45 comprises at least one opening 43 (also referred to as an eyelet) in the insertion end 41 configured to allow urine to enter a central lumen of the catheter shaft 45 and flow towards the connector 42 in the opposite end.

One of the holder parts 1, 2 of the protective sleeve arrangement is configured for attachment to a connector 42 of a urinary catheter 4. In this example, it is the first holder part 1 that is configured to (optionally releasably) attach to the connector 42. To this end, the protective sleeve arrangement 5 is threaded onto the shaft 45 of the urinary catheter, with the first holder part 1 first, and moved over the shaft 45 to the connector 42 whereby the first holder part 1 (optionally releasably) connects to the connector 42 as shown in fig. 8b.

The protective sleeve arrangement 5 is now attached to the urinary catheter 4 and is in its compacted state. The urinary catheter 4, with the protective sleeve arrangement 5 attached, can then be arranged inside an outer packaging that keeps the urinary catheter and protective sleeve arrangement 5 sterile.

In figs. 8a and 8b a complete urinary catheter 4 is first provided and subsequently the protective sleeve arrangement 5 is arranged onto the catheter 4. However, it is understood that some parts of the catheter 4 may be manufactured after the protective sleeve arrangement 5 is attached to the connector 42. For example, the one or more opening 43 at the insertion end 41 may be formed after the protective sleeve arrangement 5 is attached to the connector 42. Additionally, as the protective sleeve arrangement, in its compacted state, leaves most of, or the entire, catheter shaft 4 exposed it is envisaged that the outside of the catheter shaft 4 may be coated (e.g. with a hydrophilic coating) after the protective sleeve arrangement 5 has already been attached to the connector 42 and/or that a rounded tip at the insertion end 41 is formed after the protective sleeve arrangement 5 has already been attached to the connector 42.

The packaging containing the urinary catheter 4, with the protective sleeve arrangement 5, may then be supplied to users and when a user is to use the urinary catheter 4, the package is opened, and the urinary catheter 4 carrying the protective sleeve arrangement 5 is removed from the packaging. When the urinary catheter 4 is removed from the packaging the protective sleeve arrangement 5 is in the collapsed state wherein user may decide whether he or she wishes to use the protective sleeve arrangement 5. If the user does not want to use the protective sleeve arrangement 5 the user can perform self-catheterization without using the protective sleeve arrangement 5. As seen in fig. 8b, when the protective sleeve arrangement 5 is in the compacted state and attached to the connector 42 the catheter shaft 4 including the insertion tip 41 and eyelets 43 are exposed, allowing the user to perform self-catheterization while holding the connector 42 and/or the protective sleeve arrangement 5. Accordingly, since the protective sleeve arrangement 5 is in the collapsed state and attached to the connector 42 it does not get in the way for catheterization. Additionally, since the first holder part 1 is connected to the connector 42, and the second holder part 2 is connected to the first holder part 1 there is no risk that the holding parts 1, 2 come loose, and slide along the catheter shaft 45 during catheterization.

On the other hand, if the user wishes to use the protective sleeve arrangement 5, he or she simply pulls on the second holder part 2 to release it from the first holder part 1 whereby the tubular sleeve 3 extends to cover the catheter shaft 45, as shown in fig. 8c. When the tubular sleeve 3 is extended, the user can grab the urinary catheter 4 along the catheter shaft 45, via the tubular sleeve 3 or via the second holder part 2, which may make manipulation of the urinary catheter 4 easier before, during, and after catheterization.

In some implementations, the package is configured so as to automatically extend the protective sleeve arrangement 5 when the urinary catheter 4 is withdrawn from the package. For example, the package is configured to interact with the second holder part 2 so as to retain the second holder part 2 as the catheter 4 is pulled out (by e.g. the user grabbing the connector 42) of the package to thereby achieve extension of the protective sleeve arrangement 5. Subsequently, the second holder part 2 can be disengaged from the package to free the catheter 4 from the package. Additionally, a package configured to automatically extend the protective sleeve arrangement 5 upon withdrawal of the catheter 4 may further be configured to allow the user to remove the catheter 4 from the package without extending the protective sleeve arrangement 5. For example, upon opening the package the user may disengage (e.g. by tearing) the part of package that interacts with the second holder part 2 prior to removing the catheter 4 from the package, whereby the catheter 4 can be removed without extending the protective sleeve arrangement 5.

When the user inserts the shaft 45 of the urinary catheter 4 into the urethra, the second holder part 2 is pulled back towards the first holder part 1 and the connector 42, so as to expose progressively more of the catheter shaft 45 as it enters into the urethra. Hereby, the protective sleeve arrangement will return to an at least partly compacted state. During catheterization, urine flows into the eyelets 43 of the urinary catheter 4, through the catheter lumen inside the shaft 45, and out through the connector 42. Once the user has drained his or her bladder, the urinary catheter 4 is removed from the urethra whereby, during removal, the second holder part 2 can again be moved over the catheter shaft 45, towards the tip 41 at the insertion end, so as to cover the catheter shaft 45. When the catheter shaft 45 is removed from the urethra, there may still be traces of urine on the shaft 45 which users want to avoid getting in contact with. To this end, the tubular sleeve 3 is again used to separate the user from the catheter shaft 45. Since the user can grab the urinary catheter 4 by the catheter shaft 45, via the second holder part 2 or tubular sleeve 3 after using the catheter, it may be easier for the user to dispose of the urinary catheter 4 without touching the shaft 45.

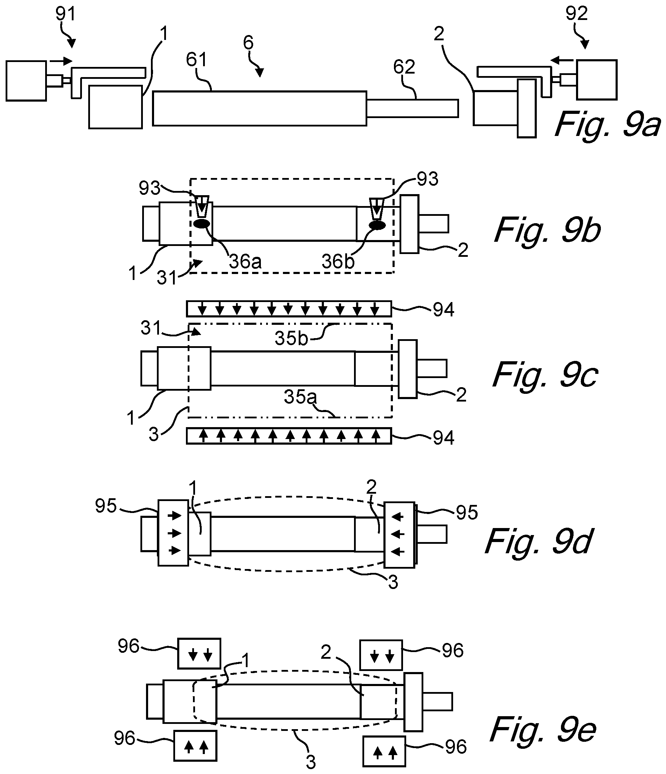

Figs. 9a-g schematically illustrate top views of components used in a manufacturing system configured to manufacture a protective sleeve arrangement. In one implementation, a manufacturing system highly suitable for mass production comprises a plurality of mandrels 6 which are moved by a conveyor between different processing stations. Each processing station performs one or more of the operations described in connection to figs. 9a-g below. For example, at a first station the holder parts 1, 2 are mounted onto the mandrel 6 using actuators 91, 92. At a second station two sheets are provided on either side of the mandrel and attached to the holder parts 1, 2 at attachment points 36a, 36b. At a third station the sheets are joined together along two joining lines 35a, 35b. The same mandrel 6 then continues to a fourth station where the tubular sleeve 3 is attached to each of the holder parts 1, 2. At a subsequent fifth station the first holder part 1 is pushed by an actuator 97 towards the second holder part 2 whereby the first and second holder part are connected to each other. At a final sixth station the protective sleeve arrangement 5 is pushed of using an actuator 97. The fifth and sixth station may be the same station or separate station and it is envisaged that the actuator which pushes the first holder part 1 into connection with the second holder part 2 and which pushes the protective sleeve arrangement 5 of the mandrel may be the same actuator or different actuators.

After the sixth station, the mandrel 6 becomes free and can be reused at the first station again. With this method, protective sleeve arrangement 5 can be manufactured efficiently at a high rate suited for mass production.

In the station depicted in fig. 9a, two actuators 91, 92 are used to push each holder part 1, 2 onto the mandrel 6. Hereby, the process of arranging the holder parts 1, 2 on the mandrel 6 may be made automatic, without human interaction. The actuators 91, 92 may be configured to align the holder parts 1, 2 with recesses and/or protrusions provided on the mandrel 6 so as to ensure that the holder parts 1, 2 are mounted on the mandrel 6 and rotationally locked relative the mandrel 6.

In the station depicted in fig. 9b the holder parts 1, 2 are provided on the mandrel 6 and two sheets 31 have been provided on either side of the mandrel 6 and holder parts 1, 2. Here, welding heads 93 are brought down onto the sheets and the holder parts 1, 2 so as to fasten each sheet to each of the holder parts 1, 2 at respective attachment points 36a, 36b. As discussed above, the welding heads 93 may be replaced with a clamping unit which temporality clamps the sheets 31 against the holder parts 1, 2 to facilitate forming of the joining line in the next station.

In the station depicted in fig. 9c, the compactable tubular sleeve 3 is formed using two welding units 94 that are brought into contact with the sheets 31 to form two elongated joining lines 35a, 35b. Optionally, the welding units 94 also cuts the sheets along the elongated joins 35a, 35b.

In the station depicted in fig. 9d, additional welding heads 95 are used to form (first) circumferential attachment regions that holds the tubular sleeve 3 onto each holder part 1, 2. As described above, the circumferential attachment regions may be larger than the attachment points 36a, 36b such that the circumferential attachment regions covers a larger portion of the perimeter of the holder parts 1, 2.

A similar procedure is performed at the station depicted in fig. 9e where welding heads 96 are used to form (second) circumferential attachment regions which are shifted 90 degrees around the circumference of the holder parts 1, 2 with respect to the first circumferential attachment regions (see also fig. 7d and fig. 7e).

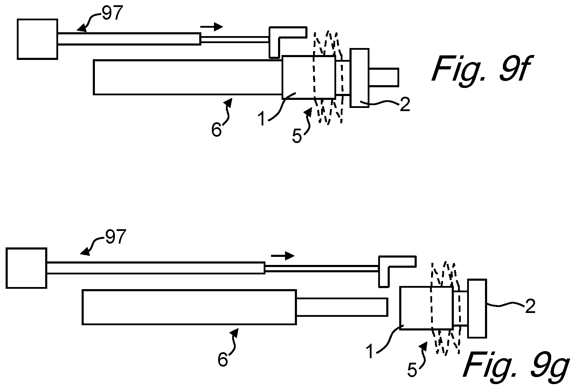

In the station depicted in fig. 9f an actuator 96 engages the first holder part 1 and moves it towards the second holder part 2 such that the holder parts 1, 2 connect to each other. Optionally, the actuator 96 continues to move the first and second holder part 1, 2 such that they come loose from the mandrel 6, as shown in fig. 9g or a separate actuator is used in a separate station to push the first and second holder part 1, 2 off the mandrel 6.

While each station depicted in the figs. 9a-g may be a separate station it is envisaged that some procedures may be performed at the same station. For example, the steps of attaching the sheets to the holder parts and forming the tubular sleeve shown in fig. 9b and 9c may be performed at substantially the same time, at the same station. Thus, any two or more of the above-discussed stations may be realized as combined stations.

In some implementations, as discussed above, it is envisaged that the tubular sleeve 3 is formed prior to arrangement of the holder parts 1, 2 on the mandrel 6. Hereby, the processing performed by the stations of figs. 9b-e may be performed first and separately, without the mandrel 6, whereafter the holder parts 1, 2 are arranged on the mandrel 6 and processing performed by the stations of fig. 9f and 9g follows.

The person skilled in the art realizes that the present invention by no means is limited to the preferred embodiments described above. On the contrary, many modifications and variations are possible within the scope of the appended claims. For example, while the first holder part in some embodiments is larger than the second holder part this may not necessarily be the case in other embodiments. For example, in some embodiments the second part is larger than the first holder part and configured to fit around the first holder part. In the claims, the word "comprising" does not exclude the presence of other elements or steps than those listed in the claim. The word "a" or "an" preceding an element does not exclude the presence of a plurality of such elements.

## Claims

1. A method for manufacturing a protective sleeve arrangement (5) for a urinary catheter (4) comprising:
providing a first holder part (1) having a channel (11) extending therethrough;
providing a second holder part (2) having a channel (21) extending therethrough;
forming, a tubular sleeve (3), the tubular sleeve (3) being flexible and attached to the first holder part (1) and to the second holder part (2);
arranging the first and second holder part (1, 2) on a mandrel (6) such that the mandrel (6) extends through the channels (11, 21) of the first and second holder part (1, 2);
moving the first holder part (1) along the mandrel, towards the second holder part (2);
releasably connecting the first holder part (1) to the second holder part (2) to form the protective sleeve arrangement (5); and
removing the protective sleeve arrangement (5) from the mandrel (6).

2. The method according to claim 1, wherein forming a tubular sleeve (3) comprises:
providing two flexible sheet layers (31, 32) on opposite sides of the holder parts (1, 2), each flexible sheet layer (31, 32) extending between the first and second holder part (1,2);
joining the two flexible sheet layers (31, 32) together along two joining lines (35a, 35b) to form the tubular sleeve (3) around the holder parts (1, 2); and
attaching respective end portions of the tubular sleeve (3) to the first holder part (1) and the second holder part (2), respectively, at circumferential attachment regions (37a, 37b, 38a, 38b).

3. The method according to claim 2, further comprising:
attaching respective end portions of the flexible sheets (31, 32) to the first holder part (1) and second holder part (2), respectively, at attachment points (36a, 36b) prior to joining the two flexible sheet layers (31, 32) to each other.

4. The method according to claim 3 when depending on claim 2, wherein the attachment points (36a, 36b) and circumferential attachment regions (37a, 37b, 38a, 38b) are at least partially overlapping.

5. The method according to any of the preceding claims, wherein the first holder part (1) comprises a first connecting feature (15) and the second holder part (2) comprises a second connecting feature (25), configured to engage the first connecting feature (15), wherein connecting the first holder part (1) and second holder part (2) comprises:
engaging the first connecting feature (15) with the second connecting feature (25).

6. The method according to claim 5, wherein the tubular sleeve (3), when connected to the first and second holder part (1, 2), covers the first and second connecting feature (15, 25), enabling the first and second connecting feature (15, 25) to connect inside the tubular sleeve (3).

7. The method according to any of the preceding claims, wherein the channel (11) of the first holder part (1) is configured to receive at least a part of the second holder part (2), and wherein connecting the first holder part (1) to the second holder part (2) comprises:
receiving the second holder part (2) at least partially inside the channel (11) of the first holder part (1).

8. The method according to any of the preceding claims, wherein the mandrel (6) comprises a first portion (61) with a first cross-sectional dimension, D₁, and a second portion (62) with a second cross-sectional dimension D₂, wherein the first cross-sectional dimension, D₁, is larger than the second cross-sectional dimension, D₂, and wherein arranging the first and second holder part (1, 2) on the mandrel (6) comprises:
arranging the first holder part (6) on the first portion (61) and arranging the second holder part (2) on the second portion (62).

9. The method according to claim 8, wherein the mandrel (6) comprises a transition portion (63) where the cross-sectional dimension changes from the first cross-sectional dimension, D₁, to the second cross-sectional dimension, D₂, and wherein arranging the second holder part (2) on the mandrel comprises:
arranging the second holder part (2) adjacent to, or preferably abutting, the transition portion (63).

10. The method according to claim 8 or claim 9, wherein the first cross-sectional dimension, D₁, is larger than an inner cross-sectional dimension of the channel (21) in the second holder part (2).

11. The method according to any of claims 8 - 10, wherein an outer cross-sectional dimension of the second holder part (2) is equal to or smaller than the first cross-sectional dimension, D₁, of the first portion (61) of the mandrel (6).

12. The method according to any of the preceding claims, wherein the mandrel (6) comprises one of an indentation (64) and a protrusion, and wherein the channel (11) of the first holder part (1) comprises the other one of an indentation and a protrusion configured to engage the indentation (64) or protrusion of the mandrel (6) so as to rotationally lock the first holder part (1) relative the mandrel (6).

13. A method for manufacturing a catheter with a protective sleeve arrangement comprising:
manufacturing a protective sleeve arrangement (5) according to any of the preceding claims;
providing a urinary catheter (4) comprising a catheter shaft (45) having an insertion end (41) and a connector (42) attached to the end of the catheter shaft (45) that is opposite the insertion end (41);
inserting the catheter shaft (45) with the insertion end (41) first through the channels (11, 21) of the first and second holder part (1, 2) of the protective sleeve arrangement (5); and
connecting the first or second holder part (1, 2) to the connector (42).

14. The method according to claim 13, further comprising:
placing the catheter (4) with the protective sleeve arrangement (5) in an outer package.

15. A manufacturing system for manufacturing a protective sleeve arrangement (5) for a urinary catheter (4), the manufacturing system comprising:
a mandrel (6),
a holder part arrangement subsystem, configured to arrange a first and second holder part (1, 2) on the mandrel (6), wherein each holder part (1, 2) comprises a channel (11, 21) through which the mandrel (6) is inserted,
a tubular sleeve forming subsystem, configured to, form a tubular sleeve (3), the tubular sleeve (3) being flexible and coupling the first holder part (1) to the second holder part (2), and
an actuator subsystem configured to slide the first holder part (1) along the mandrel (6) to releasably connect the first holder part (1) with the second holder part (2) so as to form a protective sleeve arrangement (5), and to remove the protective sleeve arrangement (5) from the mandrel (6).
